(19) Europäisches Patentamt
European Patent Office
Office européen des brevets

(11) **EP 3 750 570 A1**

(12) **EUROPEAN PATENT APPLICATION**
published in accordance with Art. 153(4) EPC

(43) Date of publication:
**16.12.2020 Bulletin 2020/51**

(21) Application number: **19751332.8**

(22) Date of filing: **06.02.2019**

(51) Int Cl.:
*A61L 31/04* (2006.01)          *A61L 31/06* (2006.01)
*C08L 71/02* (2006.01)          *C08L 5/04* (2006.01)
*C08J 7/12* (2006.01)

(86) International application number:
**PCT/KR2019/001489**

(87) International publication number:
**WO 2019/156463 (15.08.2019 Gazette 2019/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.02.2018 KR 20180014393**

(71) Applicant: **Hyunwoo Tech**
**Yangsan-si Gyeongsangnam-do 50585 (KR)**

(72) Inventors:
• **KIM, Jin Un**
  **Kimhae-si Gyeongsangnam-do 50835 (KR)**
• **KIM, Jin Tae**
  **Gyeongsan-si Gyeongsangbuk-do 38664 (KR)**

(74) Representative: **M. Zardi & Co S.A.**
**Via G. B. Pioda, 6**
**6900 Lugano (CH)**

(54) **ADHESION PREVENTING FILM HAVING EXCELLENT BONDING STRENGTH**

(57)     There is provided an adhesion prevention film having excellent adhesive property and capable of improving adhesion effects on mucous membrane, which is manufactured by the method including processes of mixing an adhesion prevention composition, which includes distilled water, polyethylene oxide (PEO), and alginate, casting the mixed adhesion prevention composition and freeze-drying, to make a sponge-type film, compressing the sponge-type film, and irradiating one or more light sources selected from radioactive rays, electron beam, gamma rays, and ultraviolet rays on the compressed film to cross-link the film, in which a sponge-type film is first made by freeze-drying after casting the adhesion prevention composition, and then, the adhesion prevention film is made by cross-linking the freeze-dried film by compressing the film, thereby to provide the adhesion prevention film showing excellence in mechanical strength, flexibility, adhesive property, cytotoxicity, biodegradability, biocompatibility, adhesion prevention property, and the like.

## Description

[Technical Field]

The present invention relates to an adhesion prevention film having excellent adhesive property and capable of improving adhesion effects on mucous membrane, which is manufactured by the method including processes of mixing an adhesion prevention composition, which includes distilled water, polyethylene oxide (PEO), and alginate, casting the mixed adhesion prevention composition and freeze-drying, to make a sponge-type film, compressing the sponge-type film, and irradiating one or more light sources selected from radioactive rays, electron beam, gamma rays, and ultraviolet rays on the compressed film to cross-link the film.

[Background Art]

Recently, efforts of applying and using biodegradable polymer in the medical treatment field such as adhesion barrier, wound covering agent, and the like are increasing.

Herein, 'adhesion' means the phenomenon that wounds and organ or tissue surrounding wounds are adhered together during the healing of the wounds formed inside the abdomen after surgical laparotomy operation when the tissue of organ is excessively exposed or bleeding, and blood is clotted.

Once such adhesion occurs, various kinds of sequelae or secondary diseases rise in a patient, and the tissue that adhesion occurs cannot engage in normal activities.

An adhesion barrier forms a physical barrier on the region where adhesion is expected to occur, in order to prevent adhesion so that adhesion is prevented from occurring between the surgical region and the normal tissues.

A film type and a gel type are much used for the adhesion barrier, but since the film type is difficult to be employed on complicated shapes of surgery region, and the gel type is dissolved or absorbed before the wound is recovered, the film type or the gel type has problems that the effect as adhesion barrier is low.

Various efforts of developing the technologies for various adhesion barriers are actively performed in order to solve the problems as above (Korean Patent Application Publication No. 10-2007-0010572, Korean Patent Registration No. 10-0552954, and Japanese Patent Application Publication No. 09-263671).

However, the films disclosed in the documents of the above cannot be remained long on wounds because the films are low in adhesive strength regarding mucous membrane, so that their adhesion prevention property is inferior.

Further, the adhesion prevention characteristics cannot be operated effectively enough because the strength of the films is low and may be warped or shrinkable easily.

[PRIOR ART DOCUMENTS]

[Patent Documents]

(Patent document 1) Korean Patent Application Publication No. 10-2007-0010572A
(Patent document 2) Korean Patent Registration No. 10-0552954B1
(Patent document 3) Japanese Patent Application Publication No. 09-263671A

[Technical Solution]

Therefore, the present invention is intended to solve the problems of the conventional technology as above, and it is required to provide an adhesion prevention composition which include distilled water, polyethylene oxide (PEO) and alginate, and improves the adhesivity (adhesive capability) on mucous membrane.

Further, the present invention is required to provide an adhesion prevention film which is manufactured by casting the adhesion prevention composition as above and free-drying to make a sponge-type film, and then, compressing the film to make the compressed film cross-linked, and shows excellent characteristics in mechanical strength, flexibility, adhesive property, cytotoxicity, biodegradability, biocompatibility, adhesion prevention property, and the like.

In order to achieve the purposes of the present invention, the present invention provides an adhesion prevention composition which includes distilled water, polyethylene oxide (PEO), and alginate, and the amount of the polyethylene oxide is from 1 to 10 parts by weight, and the amount of the alginate is from 1 to 10 parts by weight based on 100 parts by weight of distilled water.

In one embodiment of the present invention, the weight ratio of the polyethylene oxide and the alginate is 60~90:10~40.

Further, the present invention provides an adhesion prevention film which is manufactured by the processes including mixing the adhesion prevention composition as above; casting the mixed adhesion prevention composition and freeze-drying, so as to make a sponge-type film; compressing the sponge-type film; and irradiating one or more light sources selected from radioactive rays, electron beam, gamma rays, and ultraviolet rays on the compressed film, so as to cross-link the film.

In one embodiment of the present invention, the weight ratio of the polyethylene oxide and the alginate is 60~90:10~40.

Further, the present invention provides a method of manufacturing an adhesion prevention film which includes the processes of mixing the adhesion prevention composition as above; casting the mixed adhesion prevention composition and freeze-drying, to make a sponge-type film; compressing the sponge-type film; and irradiating one or more light sources selected from radioactive rays, electron beam, gamma rays, and ultraviolet rays on the compressed film to cross-link the film.

In one embodiment of the present invention, the pressure in the process of compressing is from 50 to 150$kg_f$/cm$^2$.

Further, the present invention provides various products comprising the adhesion prevention film as above, and as the products, there are adhesion barriers, wound covering agent, mask packs, pharmaceutical delivery systems, materials for tissue engineering, catheters, coating materials, separation layers, contact lenses, and the like.

[Advantageous Effects]

According to the present invention, the problems of the conventional technology are solved by providing the adhesion prevention composition which include distilled water, polyethylene oxide (PEO) and alginate, and improves the adhesive property on mucous membrane.

Further, according to the present invention, an adhesion prevention film is provided to have excellent characteristics in mechanical strength, flexibility, adhesive property, cytotoxicity, biodegradability, biocompatibility, adhesion prevention property, and the like, which is manufactured by casting the adhesion prevention composition as above and free-drying to make a sponge-type film, compressing the film, and cross-linking the compressed film.

[Best Mode]

[Mode for Invention]

The present invention will now be described more fully hereinafter based on exemplary embodiments of the present invention. It should be understood, however, that the terms and the embodiments herein disclosed are only used to explain the present invention clearly and distinguish one from other ones and help the understanding by those skilled in this art, but the spirit and scope of the present invention as defined in the appended claims should not be intended to limit the present invention to the particular forms.

Unless otherwise defined, all terms including technical and scientific terms used herein have the same meaning as commonly understood by one of ordinary skill in the art to which this present invention belongs.

The present invention is about an adhesion prevention composition which includes distilled water, polyethylene oxide (PEO), and alginate, and can improve adhesive property with respect to mucous membranes.

The composition may include 1 to 10 parts by weight of polyethylene oxide, and 1 to 10 parts by weight of alginate based on 100 parts by weight of distilled water.

The distilled water functions to control the concentration of the composition, and to swell a film in the manufacturing of the film, and to make a sponge-type film by vaporizing during freeze-drying.

The polyethylene oxide is widely used for the material of an adhesion barrier because of its excellent biodegradability, good mechanical property, and high flexibility, etc., but when used as adhesion barrier on mucous membrane, it cannot remain long at wounds, because it has low adhesivity on mucous membrane, so that its adhesion prevention property is decreased.

The weight average molecular weight of the polyethylene oxide (PEO) is preferably 10,000 to 1,000,000g/mol. In the case that the molecular weight of PEO is less than 10,000g/mol, the adhesive property and the adhesion prevention property thereof are decreased, and in the case that the molecular weight of PEO is greater than 1,000,000g/mol, its dispersibility gets inferior so that the processability is decreased.

The content of PEO is preferably 1 to 10 parts by weight based on 100 parts by weight of distilled water, and in the case that the content of PEO is less than 1 part by weight, the adhesive property and the adhesion prevention property thereof are decreased, and in the case that the content of PEO is greater than 10 parts by weight, the dispersibility and the processability thereof are decreased.

Since the alginate has excellent adhesive property, it contributes to increase the adhesive strength of the film manufactured thereby.

The weight average molecular weight of the alginate is preferably 10,000 to 100,000g/mol, and is more preferably 10,000 to 30,000g/mol. In the case that the molecular weight of alginate is less than 10,000g/mol, the adhesive property and the adhesion prevention property thereof are decreased, and in the case that the molecular weight of alginate is greater than 100,000g/mol, its dispersibility gets inferior so that the processability is decreased.

The content of the alginate is preferably 1 to 10 parts by weight based on 100 parts by weight of distilled water, and in the case that the content of the alginate is less than 1 part by weight, the adhesive property and the adhesion prevention property thereof are decreased, and in the case that the content of the alginate is greater than 10 parts by weight, the mechanical property thereof is decreased.

The weight ratio of the PEO and the alginate is preferably 60~90:10~40. If the weight ratio thereof is greater than 90:10, the adhesive property and the adhesion prevention property thereof are decreased, and if the weight ratio thereof is less than 60:40, the mechanical strength thereof comes to be decreased.

Further, the composition may additionally include pectin. Pectin is polymer of D-galacturonic acid, and includes many carboxyl groups inside molecules. This carboxyl group helps to increase the adhesive property on mucous membrane, so as to improve its adhesion prevention property.

The weight average molecular weight of the pectin is preferably 2,000 to 100,000g/mol, and in the case that the molecular weight of pectin is less than 2,000g/mol, the adhesive property and the adhesion prevention property thereof are decreased, and in the case that the molecular weight of pectin is greater than 100,000g/mol, its dispersibility gets inferior so that the processability is decreased.

The content of the pectin is preferably 1 to 5 parts by weight based on 100 parts by weight of distilled water, and when that the content of pectin is less than 1 part by weight, the effect of adding the pectin is little, while the mechanical property thereof is decreased if the content of pectin is greater than 5 parts by weight.

When the polyethylene oxide (PEO), alginate, and pectin are used, regarding the weight ratio of the PEO, the alginate and the pectin, it is preferable that the composition includes 20~50 parts by weight of alginate and 10~30 parts by weight of pectin based on 100 parts by weight of PEO. If the composition satisfies the ranges of the weight ratio as above, the adhesive property and the adhesion prevention property of the film manufactured thereby can be increased.

The composition may further include polyethylene oxide derivative, and the polyethylene oxide derivative functions to improve the adhesiveness of the adhesion prevention film on the mucous membrane which the film is attached to.

The polyethylene oxide derivative is characterized in that hydroxyl group at the terminal of the polyethylene oxide is changed to carboxyl group.

The polyethylene oxide derivative employing carboxyl group is formed by oxidizing polyethylene oxide by oxidizing agent such as $NaClO$, $KMnO_4$, $K_2Cr_2O_7$, $H_2Cr_2O_7$, $H_2CrO_4$ and the like, or formed by reacting polyethylene oxide with acid anhydride of maleic anhydride, and succinic anhydride, etc., or dicarboxylic acid of succinic acid, and adipic acid, etc.

The content of the polyethylene oxide derivative may be 1 to 10 parts by weight based on 100 parts by weight of the polyethylene oxide, and when the content of the polyethylene oxide derivative is less than 1 part by weight, the effect of adding is little, and when the content of the polyethylene oxide derivative is greater than 5 parts by weight, the mechanical property thereof is decreased.

The composition may further include polyethylene oxide-polyacrylic acid copolymer or polyethylene oxide-polymethacrylic acid copolymer, and these copolymers can improve the adhesive property of the adhesion prevention film on the mucous membrane.

The content of the added copolymer may be 1 to 10 parts by weight based on 100 parts by weight of the polyethylene oxide, and when the content of the copolymer is less than 1 part by weight, the effect of adding is little, and when the content of the copolymer is greater than 10 parts by weight, the mechanical property thereof is decreased.

The composition may further include polyethylene oxide-polylactic acid copolymer. The copolymer can improve the biodegradability of the adhesion prevention film, and improve the adhesive property of the adhesion prevention film on mucous membrane.

The content of the added copolymer may be 1 to 10 parts by weight based on 100 parts by weight of the polyethylene oxide, and when the content of the copolymer is less than 1 part by weight, the effect of adding is little, and when the content of the copolymer is greater than 10 parts by weight, the mechanical property thereof is decreased.

The present invention provides the adhesion prevention film, which is manufactured by the processes including mixing the adhesion prevention composition as above, casting the mixed adhesion prevention composition and freeze-drying, so as to make a sponge-type film, compressing the sponge-type film, and irradiating one or more light sources selected from radioactive rays, electron beam, gamma rays, and ultraviolet rays on the compressed film to cross-link the film.

Generally, an adhesion prevention film is manufactured by the cross-linking process after making a film by mixing components, and then, casting or compressing. Herein, when using the cross-linked film for the adhesion prevention film, it can be used by swelling the film with water, and then, attaching the film on a tissue region requiring adhesion prevention, or by making the film swelled by the moisture inside a human body after attaching the film on the tissue region requiring adhesion prevention.

When using the cross-linked film after swelling or when making it swelled while being used, the width, length, and height of the film are expanded upto 2 to 3 times, respectively. By such expansion, the strength and the elongation rate of the film come to be decreased, so that the film may tear easily or may be easily separated off from the adhesion prevention region.

In order to solve this problem as above, the present invention includes a process of freeze-drying after casting the composition.

In the case of making the film by a normal drying process after casting the composition, the swelled film during early time of the casting process is contracted while the moisture inside the film evaporates during the drying process, so that its width, length, and height are reduced. When using the cross-linked film manufactured as above for the adhesion prevention film, the problem of the conventional film still exists. When using the cross-linked film after swelling or when making it swelled while being used, the width, length, and height of the film are expanded upto 2 to 3 times, respectively. By such expansion, the strength and the elongation rate of the film come to be decreased, so that the film may tear easily or may be easily separated off from the adhesion prevention region.

However, when freeze-drying the film after casting the composition, even though the moisture inside the film evaporates during the drying process, the swelled film during the early time of the casting process maintains its swelled state that its width, length, and height are expanded. That is, the film can be manufactured as sponge-type film.

It is preferable to perform the freeze-drying on the film after controlling the contents of the moisture inside the film to 20 to 40 % by weight after casting the composition in the present invention. If the content of the moisture inside the film is less than 20 % by weight, adhesive property is decreased, meanwhile if the content of the moisture inside the film is greater than 40 % by weight, its mechanical strength is decreased.

The height, that is, the thickness of the sponge-type film after freeze-drying can be recovered back to its original size. Specifically, the width and the length of the film can be maintained with expanded state but its thickness only can be contracted back to its original size by compressing the freeze-dried sponge-type film with constant pressure.

The pressure in the compressing process is preferably $50\sim150kg_f/cm^2$, but if the pressure is less than $50kg_f/cm^2$, its adhesion prevention characteristics is decreased, and if the pressure is greater than $150kg_f/cm^2$, the mechanical strength of the film is decreased.

Further, the pressure of the compressing process can be divided by two steps, and specifically, in the first compressing, the film can be compressed with the pressure of $50\sim80kg_f/cm^2$, and then, the pressure in the second compressing can be controlled to $100\sim130kg_f/cm^2$. Thereby, the adhesivity and the mechanical strength of the film can be maximized by applying the pressure with two steps.

Then, by irradiating a light source on the compressed film and making it cross-linked, its cross-linking density is increased, and its mechanical property is improved, so that the decomposition velocity of the film can be controlled. Therefore, the adhesion prevention property of the film can be stably maintained while the wound is healed.

Furthermore, the adhesivity of the film can be further increased, and the adhesivity of the film on the mucous membrane and the biocompatibility can be further improved by purging acetic acid or carbon dioxide during the cross-linking of the film, and employing carboxyl group on the surface of the film in the present invention.

The radiation dose of the light source during the cross-linking is preferably 150~400kGy, and if the radiation dose of the light source is less than 150kGy, the cross-linking density of the film is decreased and the adhesivity is decreased, and if the radiation dose of the light source is greater than 400kGy, the biodegradability of the film is reduced.

When using the film manufactured by the method of the present invention for the adhesion prevention film, the film is used after swelling or the film is swelled during usage, in which the width and the length of the film are not swelled anymore because the film is already swelled at its width and length during freeze-drying, but the height only of the film is slightly expanded so that the strength of the film and the elongation rate thereof are maintained, and the film does not easily tear during usage and is not separated easily from the adhesion prevention region.

The present invention provides the method of manufacturing an adhesion prevention film, which includes mixing the adhesion prevention composition as above, casting the mixed adhesion prevention composition and freeze-drying, so as to make a sponge-type film, compressing the sponge-type film, and irradiating one or more light sources selected from radioactive rays, electron beam, gamma rays, and ultraviolet rays on the compressed film to cross-link the film.

The thickness of the adhesion prevention film of the present invention is not specifically limited, but it is preferably 50~500 $\mu$m.

Further, the present invention provides products which are manufactured with the adhesion prevention film of the present invention, and the products may include adhesion barriers, wound covering agent, mask packs, pharmaceutical delivery systems, materials for tissue engineering, catheters, coating materials, separation layers, and contact lenses.

Since the adhesion prevention film of the present invention is excellent in mechanical strength, flexibility, adhesive property, cytotoxicity, biodegradability, biocompatibility, adhesion prevention property, and the like, it can be widely used for an adhesion barrier, a wound covering agent, and the like.

Hereinafter, the present invention will now be described more fully, in which exemplary embodiments of the present invention are shown, but the present invention should not be construed as limited to the embodiments set forth herein.

(Embodiment 1)

The composition of the present invention is prepared by mixing 100 parts by weight of distilled water, 7 parts by weight of polyethylene oxide (PEO), and 3 parts by weight of alginate.

A sponge-type film is manufactured by casting the mixed composition and free-drying it.

A film is manufactured by compressing the sponge-type film with a pressure of $100 kg_f/cm^2$.

An adhesion prevention film with a thickness of $100 \mu m$ is manufactured by irradiating 250kGy of electron beam (electric current 5mA, energy 0.7MeV) on the compressed film, and making it cross-linked.

(Embodiment 2)

The film is manufacture by the same method as in the embodiment 1, except for using 0.5 parts by weight of alginate.

(Embodiment 3)

The film is manufacture by the same method as in the embodiment 1, except for using 7 parts by weight of alginate.

(Embodiment 4)

The film is manufacture by the same method as in the embodiment 1, except for additionally using 2 parts by weight of pectin.

(Embodiment 5)

The film is manufacture by the same method as in the embodiment 1, except for adding 5 parts by weight of polyethylene oxide derivative (hydroxyl group at the terminal of the polyethylene oxide is changed to carboxyl group.) based on 100 parts by weight of polyethylene oxide.

(Embodiment 6)

The film is manufacture by the same method as in the embodiment 1, except for first compressing the sponge-type film with a pressure of $70 kg_f/cm^2$, and then, secondly compressing the film with a pressure of $110 kg_f/cm^2$.

(Exemplary Embodiment 1)

The film is manufacture by the same method as in the embodiment 1, except for not using alginate.

(Exemplary Embodiment 2)

A composition is prepared by mixing 100 parts by weight of distilled water, 7 parts by weight of polyethylene oxide, and 3 parts by weight of alginate.

A film is manufactured by casting the mixed composition and naturally drying the film.

250kGy of electron beam (electric current 5mA, energy 0.7MeV) is irradiated on the film and the film is cross-linked, so as to manufacture an adhesion prevention film with a thickness of $100 \mu m$.

The mechanical property and the adhesive strength of the films manufactured by the embodiments and the exemplary embodiments are measured, so that the results are shown in Table 1 herein below.

(Mechanical Property)

The tensile strength and the tensile elastic modulus of the film are measured by using a tensile test machine (Instron 2710-105, USA), and measured with constant stretching speed of 100mm/min.

(Thermal Contraction Rate)

The length of the film is measured after cutting the film with 300mm along longitudinal direction, and 200mm along width direction, and heat-treating in the hot air oven maintained at a temperature of 150°C for 20 minutes. Then, the length of the film is measured and the thermal contraction rate is calculated by the formula as below.

$$\text{Thermal contraction rate (\%)} = (\text{length before contraction-length after}$$

$$\text{contraction})/(\text{length before contraction}) \times 100$$

(Adhesive Strength)

The adhesiveness test of the tissue of beef entrails is performed by using a tensile test machine (Instron 4464, UK).
The manufactured film is prepared with 1 inch of width and 7 inches of length, and then, folded. The folded film is fixed with above grip by attaching the both ends by using 1/2 inch mast tape to be a water drop shape.
The film and the beef entrails (small intestine) are made to contact for 5 min. after cutting the beef entrails (small intestine) with 1 square inch, and fixing the cut tripe part at below grip, and then, moving cross head downward.
Maximum power necessary to separate the film and the tripe is measured.

[Table 1]

| Division | Embodiments | | | | | | Exemplary Embodiments | |
|---|---|---|---|---|---|---|---|---|
| | 1 | 2 | 3 | 4 | 5 | 6 | 1 | 2 |
| Tensile Strength (MPa) | 0.35 | 0.28 | 0.29 | 0.36 | 0.36 | 0.39 | 0.23 | 0.25 |
| Tensile Elastic Modulus (MPa) | 1.9 | 1.8 | 2.0 | 2.2 | 1.9 | 2.3 | 1.5 | 1.4 |
| Thermal Contraction Rate (%) | 3.6 | 3.9 | 4.0 | 3.1 | 3.3 | 2.9 | 7.1 | 6.9 |
| Adhesive Strength (cN) | 6.9 | 6.3 | 6.4 | 7.0 | 7.3 | 7.6 | 5.6 | 5.5 |

From the results of Table 1, the adhesion prevention films in embodiments 1-6 of the present invention show excellent results in mechanical strength, thermal contraction rate, and adhesiveness on the mucous membrane, so that it can be understood that the film of the present invention can be stably used for an adhesion barrier, wound covering agent, and the like. Particularly, the film in embodiment 6 shows the most excellent properties.
In the meantime, the films of exemplary embodiments 1 and 2 show inferior in the mechanical strength, thermal contraction rate, and adhesiveness on the mucous membrane compared with those of embodiments 1-6 of the present invention.

**Claims**

1. An adhesion prevention composition comprising:

    distilled water;
    polyethylene oxide (PEO); and
    alginate,
    wherein the amount of the polyethylene oxide is from 1 to 10 parts by weight, and the amount of the alginate is from 1 to 10 parts by weight based on 100 parts by weight of distilled water.

2. The adhesion prevention composition according to claim 1,
    wherein the weight ratio of the polyethylene oxide and the alginate is 60~90:10~40.

3. An adhesion prevention film manufactured by process comprising:

mixing the adhesion prevention composition of claim 1;
casting the mixed adhesion prevention composition and freeze-drying to make a sponge-type film;
compressing the sponge-type film; and
irradiating one or more light sources selected from radioactive rays, electron beam, gamma rays, and ultraviolet rays on the compressed film to cross-link the film.

4. The adhesion prevention film according to claim 3,
wherein the weight ratio of the polyethylene oxide and the alginate is 60~90:10~40.

5. A method of manufacturing an adhesion prevention film comprising:

mixing the adhesion prevention composition of claim 1;
casting the mixed adhesion prevention composition and freeze-drying to make a sponge-type film;
compressing the sponge-type film; and
irradiating one or more light sources selected from radioactive rays, electron beam, gamma rays, and ultraviolet rays on the compressed film to cross-link the film.

6. A method of manufacturing an adhesion prevention film according to claim 5,
wherein the pressure in the compressing is from 50 to $150kg_f/cm^2$.

7. A product comprising the adhesion prevention film of claim 3,
wherein the product comprises adhesion barriers, wound covering agent, mask packs, pharmaceutical delivery systems, materials for tissue engineering, catheters, coating materials, separation layers, and contact lenses.

## INTERNATIONAL SEARCH REPORT

| International application No. |
|---|
| PCT/KR2019/001489 |

| A. | CLASSIFICATION OF SUBJECT MATTER |
|---|---|

*A61L 31/04(2006.01)i, A61L 31/06(2006.01)i, C08L 71/02(2006.01)i, C08L 5/04(2006.01)i, C08J 7/12(2006.01)i*

According to International Patent Classification (IPC) or to both national classification and IPC

| B. | FIELDS SEARCHED |
|---|---|

Minimum documentation searched (classification system followed by classification symbols)

A61L 31/04; A61F 13/02; A61K 9/00; A61L 15/08; A61L 15/28; A61L 15/32; C08J 3/24; C08J 5/18; A61L 31/06; C08L 71/02; C08L 5/04; C08J 7/12

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: antiadhesion, polyethylene oxide, PEO, alginate

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KIM, Duckil et al. Characterization of Anti-Adhesion Properties of Alginate/Polyethylene Oxide Film to Reduce Postsurgical Peritoneal Adhesions. Science of Advanced Materials. 2017, vol. 9, pages 1669-1677 See page 1670, right column, 2.2. section; page 1674, right column, 4. section; page 1676, right column, lines 14-17. | 1,2 |
| Y | | 3-7 |
| Y | KR 10-1607849 B1 (RESEARCH COOPERATION FOUNDATION OF YEUNGNAM UNIVERSITY) 31 March 2016 See abstract; paragraph [0056]. | 3-7 |
| A | KR 10-2016-0009724 A (GENEWEL CO., LTD.) 27 January 2016 See the entire document. | 1-7 |
| A | KR 10-2006-0134346 A (KOREA ATOMIC ENERGY RESEARCH INSTITUTE) 28 December 2006 See the entire document. | 1-7 |
| A | KR 10-2006-0124927 A (GENIC CO., LTD.) 06 December 2006 See the entire document. | 1-7 |

| ☐ Further documents are listed in the continuation of Box C. | ☒ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 MAY 2019 (10.05.2019) | **13 MAY 2019 (13.05.2019)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea Facsimile No. +82-42-481-8578 | Telephone No. |

Form PCT/ISA/210 (second sheet) (January 2015)

INTERNATIONAL SEARCH REPORT
Information on patent family members

| International application No. |
| --- |
| **PCT/KR2019/001489** |

| Patent document cited in search report | Publication date | Patent family member | Publication date |
| --- | --- | --- | --- |
| KR 10-1607849 B1 | 31/03/2016 | WO 2017-082446 A1 | 18/05/2017 |
| KR 10-2016-0009724 A | 27/01/2016 | CN 106535946 A<br>JP 2017-522162 A<br>KR 10-1649792 B1<br>WO 2016-010330 A1 | 22/03/2017<br>10/08/2017<br>22/08/2016<br>21/01/2016 |
| KR 10-2006-0134346 A | 28/12/2006 | None | |
| KR 10-2006-0124927 A | 06/12/2006 | KR 10-0676937 B1 | 02/02/2007 |

Form PCT/ISA/210 (patent family annex) (January 2015)

**EP 3 750 570 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020070010572
- KR 100552954
- JP 9263671 A
- KR 1020070010572 A
- KR 100552954 B1